# EUROPÄISCHE PATENTSCHRIFT

(11) **EP 2 757 148 B1**
(45) Veröffentlichungstag und Bekanntmachung des Hinweises auf die Patenterteilung: **16.12.2015**
(21) Anmeldenummer: 13000264.5
(22) Anmeldetag: 18.01.2013
(51) Int. Cl.: C12M 1/00, C12M 1/24

(54) **Kulturflasche**
Culture bottle
Bouteille pour culture

(43) Veröffentlichungstag der Anmeldung: 23.07.2014
(73) Patentinhaber: EPPENDORF AG, 22339 Hamburg (DE)
(72) Erfinder: Seippel, Martin, 22949 Ammersbek (DE)
(74) Vertreter: Hauck Patentanwaltspartnerschaft mbB

(56) Entgegenhaltungen:
- EP-A2- 0 743 362
- WO-A1-2006/019836
- US-A- 4 334 028

## Beschreibung

Die Erfindung bezieht sich auf eine Kulturflasche zum Kultivieren von Zellen, Geweben oder Mikroorganismen.

Kulturflaschen (auch: "Zellkulturflaschen" genannt) werden im Labor zur Anzucht und Gewinnung von Zellen, Geweben oder Mikroorganismen verwendet. Sie haben einen flachen, im Wesentlichen rechteckigen Flaschenkörper mit Boden-, Deck- und Seitenwänden, die eine Kulturkammer begrenzen. Die Oberseite der Bodenwand ist eine Wachstumsfläche für Zellen, Gewebe oder Mikroorganismen. Ein Flaschenhals mit einem Deckel steht von einer schmalen vorderen Seitenwand nach außen vor. Bei der Anwendung liegen Kulturflaschen üblicherweise mit ihrer großen Bodenwand auf einem horizontalen Untergrund auf. Der Abstand zwischen der ebenen Bodenwand und der ebenen Deckwand der Kulturflasche wird als Höhe oder Dicke bezeichnet. Aufeinandergestapelt sind Kulturflaschen platzsparend in einem Inkubator anordenbar, der das Klima für das Wachstum der Zellen, Gewebe oder Mikroorganismen bereitstellt. Der Deckel ist vielfach aus einer Schließ- in eine Belüftungsstellung bringbar oder als Filterdeckel ausgeführt, um einen Gasaustausch zwischen Inkubator und Kulturkammer im Flaschenkörper zu gewährleisten.

Zellen, Gewebe oder Mikroorganismen werden mittels einer serologischen Pipette, eines Schabers oder einer Kanüle aus der Kulturflasche entfernt, die von außen durch den Flaschenhals in die Kulturkammer hinein und an die Wachstumsfläche herangeführt wird. Nährmedium wird ebenfalls mittels der serologischen Pipette durch den Flaschenhals hindurch in die Kulturflasche hineinpipettiert oder herauspipettiert. Diese Arbeiten sind mühselig, insbesondere wenn sie in einer Sterilbank durchgeführt werden und der Anwender Schutzkleidung trägt.

Der Flaschenhals ist in einem Abstand von der Bodenwand mit dem Rand einer Öffnung in der Seitenwand verbunden, um zu vermeiden, dass bei geöffneter Kulturflasche Flüssigkeit aus der Kulturkammer herausschwappt. Die Öffnung ist somit unten durch den Abstand des Flaschenhalses von der Bodenwand und oben durch die geradlinige Fügekante begrenzt, entlang der der obere Rand der Seitenwand mit der Deckwand verbunden ist. Zur Erleichterung des Zugangs zur Wachstumsfläche mittels einer serologischen Pipette oder eines Schabers sind Kulturflaschen bekannt, die einen zur Wachstumsfläche spitzwinklig geneigten Flaschenhals aufweisen. Das Heranführen der serologischen Pipette oder des Schabers insbesondere an die hinteren Ecken der Wachstumsfläche ist durch die Kante am oberen Rand der Öffnung erschwert. Das Entlanggleiten der serologischen Pipette oder des Schaber an dieser Kante ist nämlich mit einem höheren Widerstand als das Gleiten entlang der glatten Fläche des zylindrischen Flaschenhalses verbunden.

Die EP 0 743 362 B1 beschreibt eine Laborflasche mit einem Flaschenkörper, der eine kreisförmige Öffnung in einer vorderen Seitenwand hat. Ein langgestreckter, kreisringförmiger Flaschenhals mit einer innen und außen zylindrischen Wand erstreckt sich vom Rand der Öffnung aus nach außen. Ein Teil der Innenseite des Flaschenhalses ist oberhalb der Deckwand des Flaschenkörpers angeordnet. Der Flaschenhals weist eine herabhängende Füllwand auf, die den Spalt zwischen der Innenseite des Flaschenhalses und der Deckwand nahe der vorderen Seitenwand schließt. Bei dieser Laborflasche wird der Zugang zur Wachstumsfläche durch die Füllwand am oberen Rand der Öffnung begrenzt.

Die WO 2006/099127 A1 beschreibt eine Zellkulturflasche, die einen Flaschenkörper aufweist, der als Zellkulturkammer dient. Die Zellkulturkammer ist begrenzt durch eine Bodenschale mit einer starren Oberfläche und durch eine Deckwand, wobei die Bodenschale und die Deckwand an Seiten- und Stirnwänden miteinander verbunden sind. An mindestens einer Öffnung ist ein schräg geneigter Flaschenhals vorhanden und mindestens ein Schraubdeckel ist auf die Öffnung des Flaschenhalses ausgerichtet. Neben dem geneigten Flaschenhals weisen die Bodenwand und die Deckwand zum Flaschenhals hin ansteigende und abfallende Einziehungen auf, damit keine Flüssigkeit in den Ecken gefangen bleibt. Der geneigte Flaschenhals verbessert den Zugang zur Zellkulturkammer durch eine Pipette, einen Schaber oder eine Kanüle. Zudem stellen die ansteigenden und abfallenden Einziehungen einen Freiraum für den Deckel zur Verfügung, sodass dieser nicht mit einer benachbarten Zellkulturflasche in einem Stapel zusammenstößt. Zur Vergrößerung der Zellkulturfläche ist der Flaschenhals bevorzugt an einer Ecke der Zellkulturflasche angeordnet.

Die WO2006/019836 A1 beschreibt eine Kulturflasche mit einem Oberteil, das komplett abgenommen werden kann, um das ganze Volumen der Kulturflasche zu erreichen. Die US 4,334,028 A beschreibt eine Kulturflasche mit einem aufbrechbaren Bereich in der Deckwand für den Zugang zum Inneren zur Kulturflasche.

Davon ausgehend liegt der Erfindung die Aufgabe zugrunde, eine Kulturflasche zur Verfügung zu stellen, die den Zugang zur Wachstumsfläche auf der Bodenwand für eine serologische Pipette, einen Schaber, eine Kanüle oder eine andere langgestreckte Vorrichtung zum Zugeben oder Entnehmen von Material erleichtert.

Die Aufgabe wird durch eine Kulturflasche mit den Merkmalen von Anspruch 1 gelöst. Vorteilhafte Ausgestaltungen der Kulturflasche sind in Unteransprüchen angegeben.

Die erfindungsgemäße Kulturflasche hat
- eine Kulturkammer in einem Flaschenkörper, der eine ebene Bodenwand mit einer Wachstumsfläche an der Oberseite, eine ebene Deckwand in einem Abstand von der Bodenwand und den Abstand zwischen den Rändern der Bodenwand und der Deckwand überbrückende Seitenwände aufweist,
- eine Öffnung, die in einem Abstand von der Bodenwand in aneinander angrenzenden Bereichen einer Seitenwand und der ebenen Deckwand vorhanden ist und einen unteren Öffnungsabschnitt (13.1) in der Seitenwand (10) und einen oberen Öffnungsabschnitt (13.2) in der Deckwand (4) hat, und
- einen hohlzylindrischen Flaschenhals, der mit dem Rand des unteren Öffnungsabschnittes und dem Rand des oberen Öffnungsabschnittes verbunden und spitzwinklig zur Wachstumsfläche ausgerichtet ist, sodass die serologische Pipette, ein Schaber, eine Kanüle oder eine andere langgestreckte Vorrichtung zum Zugeben oder Entnehmen von Material von außen durch den Flaschenhals hindurch insbesondere an die hinteren Ecken der Wachstumsfläche ansetzbar ist.

Bei der erfindungsgemäßen Kulturflasche ist die Öffnung des Flaschenkörpers in zwei aneinander angrenzenden Bereichen einer Seitenwand und der Deckwand ausgebildet. Die Öffnung hat also zwei Öffnungsabschnitte, wobei ein unterer Öffnungsabschnitt in einer Seitenwand und ein oberer Öffnungsabschnitt in der Deckwand angeordnet ist. Der spitzwinklig zur Wachstumsfläche geneigte hohlzylindrische Flaschenhals ist mit dem Rand der Öffnung verbunden, d.h. mit dem Rand des unteren und dem Rand des oberen Öffnungsabschnittes. Dadurch, dass der hohlzylindrische Flaschenhals mit dem Rand der Öffnung verbunden ist, ist sein innerer Querschnitt nicht teilweise durch den Rand der Öffnung abgedeckt. Eine serologische Pipette, ein Schaber oder eine andere langgestreckte Vorrichtung zum Zugeben oder Entnehmen von Material ist durch den Flaschenhals und die Öffnung in der Seitenwand und der Deckwand hindurch in die Kulturkammer im Flaschenkörper einführbar und auf die Wachstumsfläche und die angrenzenden Innenflächen der hinteren und seitlichen Seitenwände ansetzbar. Dies ist einfacher als bei herkömmlichen Kulturflaschen, insbesondere weil die Öffnung den inneren Querschnitt des Flaschenhalses oben nicht durch eine geradlinige Fügekante abdeckt. Infolgedessen wird der Zugang zur Wachstumsfläche erleichtert, insbesondere an den hinteren Ecken der Wachstumsfläche gegenüber dem Flaschenhals. Die Kulturflasche kann so ausgebildet sein, dass mittels einer serologischen Pipette, eines Schabers, einer Kanüle oder einer anderen langgestreckten Vorrichtung die Innenflächen der hinteren Seitenwand gegenüber dem Flaschenhals und der beiden dazu benachbarten längsseitigen Seitenwände besser erreichbar sind, wodurch das Ausrichten der langgestreckten Vorrichtung auf die beiden hinteren Ecken der Zellkulturflasche erleichtert wird.

Der Flaschenhals ist mindestens an der Öffnung hohlzylindrisch, um den Zugang zur Wachstumsfläche für eine langgestreckte Vorrichtung zum Zugeben und Entnehmen von Material zu erleichtern. In einem Abstand von der Öffnung kann der Flaschenhals auch eine von einem Hohlzylinder abweichende Form aufweisen. Bevorzugt ist der Flaschenhals überall hohlzylindrisch.

Gemäß einer weiteren Ausgestaltung hat der Flaschenhals mehrere hohlzylindrische Flaschenhalsteile. Die hohlzylindrischen Flaschenhalsteile sind gemäß einer weiteren Ausgestaltung in Längsrichtung des Flaschenhalses hintereinander angeordnet. Gemäß einer weiteren Ausgestaltung weisen die hohlzylindrischen Flaschenhalsteile verschiedene Innendurchmesser auf und/oder verschiedene Außendurchmesser. Gemäß einer weiteren Ausgestaltung ist ein hohlzylindrischer Flaschenhalsteil mit einem größeren Innendurchmesser weiter hinten als ein hohlzylindrisches Flaschenhalsteil mit einem kleineren Innendurchmesser angeordnet. Somit erweitert sich der Flaschenhals generell von vorn nach hinten. Dies ist vorteilhaft insbesondere für das Erreichen der hinteren Ecken mittels einer langgestreckten Vorrichtung für das Zugeben oder Entnehmen von Material. Gemäß einer weiteren Ausgestaltung hat ein aus mehreren Bauteilen zusammengesetzter Flaschenhals Flaschenhalsteile, die verschiedene Innendurchmesser aufweisen und/oder verschiedene Außendurchmesser.

Gemäß einer Ausgestaltung weist die Zellkulturflasche ein Flaschenunterteil und ein Flaschenoberteil auf, wobei das Flaschenunterteil die Bodenwand, die Seitenwände und ein mit einer Seitenwand verbundenes erstes Flaschenhalsteil, das Flaschenoberteil, die Deckwand und ein mit der Deckwand verbundenes zweites Flaschenhalsteil aufweist und das Flaschenunterteil und das Flaschenoberteil an benachbarten Rändern abdichtend miteinander verbunden sind. Das Flaschenunterteil und das Flaschenoberteil sind vorteilhaft aus Kunststoff durch Spritzgießen und ein anderes geeignetes Verfahren herstellbar. Flaschenunterteil und Flaschenoberteil sind vorzugsweise jeweils einteilig durch Spritzgießen hergestellt. Vorzugsweise sind Flaschenunterteil und Flaschenoberteil an den benachbarten Rändern abdichtend unlösbar miteinander verbunden. Alternativ sind Flaschenunterteil und Flaschenoberteil an den benachbarten Rändern lösbar miteinander verbunden, beispielsweise durch eine Schnappverbindung, wobei zur Abdichtung ein weichelastisches Dichtelement zwischen den benachbarten Rändern angeordnet sein kann.

Gemäß einer weiteren Ausgestaltung ist das erste Flaschenhalsteil ein Hohlzylinder, der in einem unteren Randabschnitt eines stirnseitigen Randes, der in einer zur Wachstumsfläche senkrechten Fläche umläuft, mit dem Rand eines unteren Öffnungsabschnitts der Öffnung in der Seitenwand verbunden ist, und bei der das zweite Flaschenhalsteil ein Hohlzylindersegment ist, welches an einem stirnseitigen Rand mit einem oberen Randabschnitt des stirnseitigen Randes des ersten Flaschenhalsteils verbunden ist und an seinem längsseitigen Rand mit dem Rand eines sich zum ersten Flaschenhalsteil hin erweiternden oberen Öffnungsabschnitts in der Deckwand verbunden ist. Diese Ausgestaltung ist insbesondere vorteilhaft für die Herstellung von Flaschenunterteil und Flaschenoberteil durch Spritzgießen.

Gemäß einer weiteren Ausgestaltung hat das Hohlzylindersegment einen größeren Innendurchmesser als der Hohlzylinder. Gemäß einer weiteren Ausgestaltung ist das Hohlzylindersegment an seinem vorderen Rand am Innenumfang mit dem Außenumfang am hinteren Rand des Hohlzylinders verbunden.

Ferner ist diese Ausgestaltung vorteilhaft für das Verbinden von Flaschenunterteil und Flaschenoberteil.

Gemäß einer weiteren Ausgestaltung ist die Verbindung zwischen Flaschenunterteil und Flaschenoberteil eine Ultraschall-Schweißverbindung oder eine Infrarot-Schweißverbindung oder eine Klebeverbindung oder eine Rastverbindung.

Beim Ultraschall-Schweißen werden die Materialien durch hochfrequente Schwingungen der Fügepartner (Flaschenunterteil und Flaschenoberteil) zueinander aufgeschmolzen und dabei miteinander gefügt. Die Schwingung wird von einem Ultraschallgenerator erzeugt und mittels eines Werkzeugs (sog. "Sonotrode") in einen Fügepartner eingeleitet. Die stoffschlüssige Verbindung zwischen dem Flaschenunterteil und dem Flaschenoberteil hat eine hohe Festigkeit. Das Ultraschallschweißen ist insbesondere vorteilhaft anwendbar, wenn Flaschenunterteil und Flaschenoberteil aus Polystyrol hergestellt sind.

Beim Infrarotschweißen werden die Fügepartner durch Wärmestrahlung in den Fügezonen angeschmolzen. Hierfür wird beispielsweise ein Infrarotstrahler neben den Fügezonen zwischen dem Flaschenunterteil und dem Flaschenoberteil platziert. Nach dem Anschmelzen der Fügezonen werden die Fügepartner gefügt. Dies geschieht beispielsweise nach dem Entfernen des Infrarotstrahlers durch Zusammenpressen von Flaschenunterteil und Flaschenoberteil.

Beim Kleben wird ein Klebstoff zwischen den Fügepartnern eingebracht. Ferner ist darauf zu achten, dass der Klebstoff oder darin enthaltene Lösungsmittel unschädlich für die zu kultivierenden Zellen, Gewebe oder Mikroorganismen sind. Zusätzlich oder alternativ wird die Klebeverbindung gegenüber der Kulturkammer abgekapselt, beispielsweise indem Klebeverbindung durch eine Dichtung von der Kulturkammer abgetrennt wird.

Gemäß einer weiteren Ausgestaltung ist die Ultraschall-Schweißverbindung zwischen Flaschenunterteil und Flaschenoberteil hergestellt durch Einschweißen eines am Rand des Flaschenoberteils umlaufend nach unten vorstehenden Energierichtungsgebers in eine am oberen Rand des Flaschenunterteils umlaufende und nach oben offene Nut. Das Einschweißen des Energierichtungsgebers in die Nut erfolgt vorzugsweise mittels einer an die Oberseite des Flaschenoberteils angesetzten Sonotrode. Durch den Energierichtungsgeber wird die von der Sonotrode in das Flaschenoberteil eingeleitete Energie in den Grund der Nut eingeleitet. Die Schmelze sammelt sich in der Nut und füllt sie auf. Nach dem Erstarren der Schmelze sind Flaschenunterteil und Flaschenoberteil dauerhaft und abdichtend miteinander verbunden. Die Konturen von Nut und Energierichtungsgeber sind dann nicht mehr oder nur noch teilweise vorhanden bzw. durch die stoffschlüssige Verbindung zwischen Flaschenoberteil und Flaschenunterteil ersetzt.

Gemäß einer bevorzugten Ausgestaltung hat vor dem Verschweißen der Energierichtungsgeber die Form einer Rippe mit einem dreieckigen Querschnitt und einer Spitze des Dreiecks am unteren Ende. Weiterhin vorzugsweise beträgt der Winkel zwischen den Seitenflächen des Energierichtungsgebers an der Spitze 50° bis 70°, weiterhin vorzugsweise etwa 60°. Vorzugsweise ist vor dem Verschweißen seitlich zwischen dem Energierichtungsgeber und den Seitenwänden der Nut ein kleiner Freiraum vorhanden.

Gemäß einer weiteren Ausgestaltung weist das Flaschenoberteil am Rand eine nach oben vorstehende, ununterbrochen oder unterbrochen umlaufende erste Rippe auf. Die erste Rippe ist vorteilhaft für das Aufsetzen einer Sonotrode nutzbar. Vorzugsweise ist die erste Rippe hierfür in senkrechter Richtung über dem Energierichtungsgeber angeordnet. Die Energie kann von der Sonotrode über die erste Rippe durch die Deckwand direkt in den Energierichtungsgeber eingeleitet werden. Zudem wird durch das Ansetzen der Sonotrode an die erste Rippe eine Beschädigung der Deckwand vermieden, die sich bei flächigem Ansetzen der Sonotrode auf die Deckwand ergeben kann. Ferner kann die erste Rippe als Führungselement dienen, die eine auf die Kulturflasche aufgesetzte, weitere Kulturflasche an einer seitlichen Verlagerung hindert.

Gemäß einer weiteren Ausgestaltung steht von der Bodenwand eine ununterbrochen oder unterbrochen umlaufende zweite Rippe mit einer ebenen Unterseite nach unten vor. Die zweite Rippe bildet mit der Unterseite eine Aufstandsfläche. Ferner kann die zweite Rippe als Führungselement dienen, welche die Kulturflasche in einer seitlichen Verlagerung hindert, wenn sie auf eine Kulturflasche aufgesetzt ist. Hierfür ist die zweite Rippe bevorzugt seitlich bezüglich der ersten Rippe versetzt angeordnet.

Gemäß einer anderen Ausgestaltung ist die Infrarot-Schweißverbindung zwischen Flaschenunterteil und Flaschenoberteil hergestellt durch Aufschmelzen und Zusammenpressen einer am Rand des Flaschenoberteil umlaufend nach unten vorstehenden dritten Rippe und einer am oberen Rand des Flaschenunterteil umlaufend nach oben vorstehenden vierten Rippe. Die dritten und vierten Rippen haben vorzugsweise einen Rechteckquerschnitt und werden an streifenförmigen Stirnflächen miteinander verschweißt. Durch diese Geometrie wird das gezielte Erhitzen und miteinander Verschweißen der Fügezonen gefördert.

Gemäß einer weiteren Ausgestaltung weist der Flaschenhals mindestens ein Außengewinde und/oder mindestens ein Schnappelement für einen Deckel auf. Mittels des Außengewindes und/oder des Schnappelements ist ein mit komplementären Elementen versehener Deckel lösbar und/oder von einer Schließstellung in eine Belüftungsstellung verstellbar an der Zellkulturflasche anbringbar.

Gemäß einer weiteren Ausgestaltung verjüngt sich die Zellkulturflasche zum Flaschenhals hin. Hierdurch wird das Ausgießen von Medium aus der Zellkulturflasche erleichtert. Zudem werden durch die Verjüngung der Zellkulturflasche Bereiche einer Wachstumsfläche vermieden, die mittels einer serologischen Pipette oder einer anderen Zuführ- und Entnahmevorrichtung nicht erreichbar sind.

Gemäß einer weiteren Ausgestaltung ist zwischen Bodenwand und Seitenwand, an die der Flaschenhals angesetzt ist, eine spitzwinklig zur Bodenwand geneigte Schrägwand vorhanden. Die Schrägwand erleichtert das Ausgießen von Medium aus der Kulturflasche und das Verlagern der langgestreckten Vorrichtung für die Zufuhr oder Entnahme von Material.

Gemäß einer weiteren Ausgestaltung ist der Flaschenhals zylindrisch. Gemäß einer bevorzugten Ausgestaltung ist er kreiszylindrisch Die zylindrische und die kreiszylindrische Form begünstigt ein widerstandsarmes Verlagern einer serologischen Pipette oder einer anderen langgestreckten Vorrichtung im Flaschenhals.

Gemäß einer weiteren Ausgestaltung ist der Übergang zwischen dem Flaschenhals und dem Inneren der Kulturkammer ganz oder teilweise glatt und/oder gerundet. Hierdurch wird das Verlagern der serologischen Pipette oder eines Schabers oder einer anderen langgestreckten Vorrichtung für die Zufuhr oder Entnahme von Material im Flaschenhals erleichtert.

Gemäß einer weiteren Ausgestaltung ist die ebene Deckwand parallel zur ebenen Bodenwand ausgerichtet.

Gemäß einer weiteren Ausgestaltung sind die Oberseite und die Unterseite der Kulturflasche so aufeinander abgestimmt, dass mehrere gleiche Zellkulturflaschen aufeinander stapelbar sind.

Gemäß einer weiteren Ausgestaltung hat die Seitenwand, an die der Flaschenhals angesetzt ist, unterhalb des Flaschenhalses eine bogenförmige Aussparung, die geeignet ist, den von der Deckwand vorstehenden Bereich des Flaschenhalses einer darunter angeordneten, gleichen Kulturflasche aufzunehmen. Hierdurch wird das Aufeinanderstapeln von Kulturflaschen ermöglicht.

Gemäß einer weiteren Ausgestaltung ist die Kulturflasche durch Spritzgießen hergestellt. Vorzugsweise besteht die Kulturflasche aus einem Flaschenunterteil und einem Flaschenoberteil, die gesondert durch Spritzgießen hergestellt und danach abdichtend miteinander verbunden sind.

Gemäß einer weiteren Ausgestaltung ist die Kulturflasche aus Polystyrol oder aus einem anderen Kunststoff hergestellt.

Die Erfindung wird nachfolgend anhand der anliegenden Zeichnungen eines Ausführungsbeispiels näher erläutert. In den Zeichnungen zeigen:
- Fig. 1: die Kulturflasche in einer Seitenansicht;
- Fig. 2: die Kulturflasche in einem Längsschnitt;
- Fig. 3: die Kulturflasche in einer vergrößerten Detailansicht III von Fig. 2;
- Fig. 4: die Kulturflasche in einer Draufsicht;
- Fig. 5: die Kulturflasche in einer Rückansicht;
- Fig. 6: Flaschenunterteil und Flaschenoberteil der Kulturflasche vor dem Zusammenfügen in einem teilweisen Längsschnitt;
- Fig. 7: Flaschenunterteil und Flaschenoberteil vor dem Zusammenfügen in einem vertikalen Teilschnitt aus einer Perspektive schräg von unten und von einer Seite;
- Fig. 8: Flaschenunterteil und Flaschenoberteil vor dem Zusammenfügen in einem Vertikalschnitt in einer Perspektive schräg von oben und von einer anderen Seite;
- Fig. 9: eine alternative Ausgestaltung der Fügezonen von Flaschenunterteil und Flaschenoberteil für eine Infrarot-Schweißverbindung vor dem Zusammenfügen in einem Vertikalschnitt;
- Fig. 10: die Kulturflasche in einer Perspektivansicht schräg von vom und von der Seite;
- Fig. 11: eine herkömmliche Kulturflasche in einer Perspektivansicht schräg von vom und von der Seite;
- Fig. 12a-c: mittels einer serologischen Pipette erreichbare Flächen in der erfindungsgemäßen Kulturflasche in einer Draufsicht (Fig. 12a); ein einem Schnitt entlang der Linie b-b von Fig. 12a (Fig. 12b) und in einem Schnitt entlang der Linie c-c von Fig. 12a (Fig. 12c);
- Fig. 13a-c: mittels einer serologischen Pipette erreichbare Flächen in der herkömmlichen Kulturflasche in einer Draufsicht (Fig. 13a), in einem Schnitt entlang der Linie b-b von Fig. 13a (Fig. 13b) und in einem Schnitt entlang der Linie c-c von Fig. 13a (Fig. 13c);

In der vorliegenden Anmeldung beziehen sich die Angaben "oben", "oberhalb", "unten" und "unterhalb" auf die Ausrichtung einer Kulturflasche der Bodenwand auf einem horizontalen Untergrund und der Deckwand oberhalb der Bodenwand. Die Angaben "vorn" und "hinten" beziehen sich auf eine Kulturflasche, die mit dem Flaschenhals dem Betrachter näher ist als mit dem Flaschenkörper.

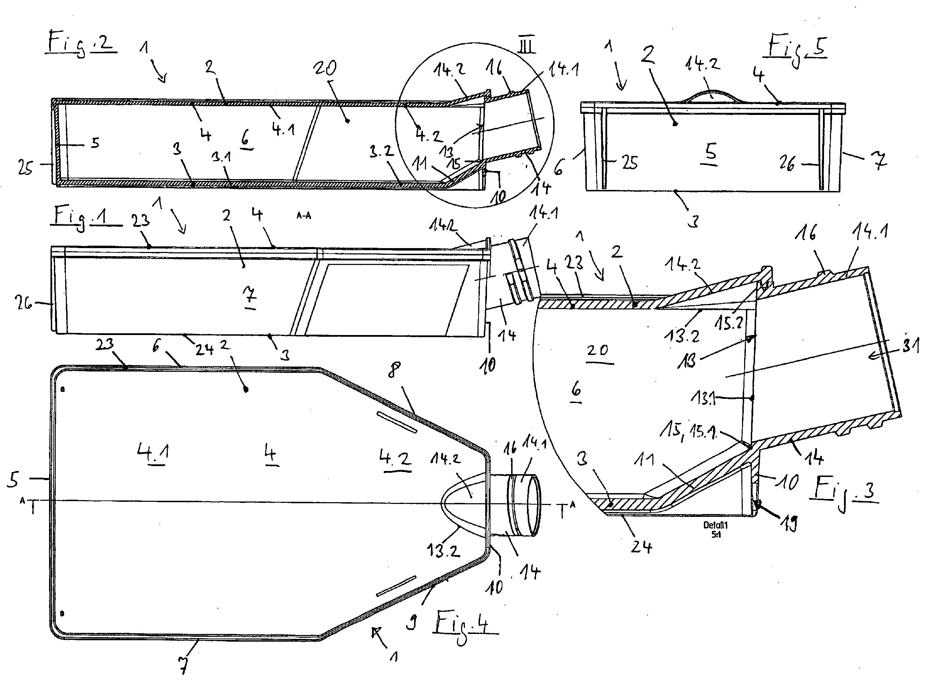

Gemäß Fig. 1 bis 5 hat eine erfindungsgemäße Kulturflasche 1 einen Flaschenkörper 2 mit einer im Wesentlichen ebenen Bodenwand 3 und einer im Wesentlichen ebenen Deckwand 4, die parallel zueinander ausgerichtet sind. Die Bodenwand 3 und die Deckwand 4 haben jeweils einen im Wesentlichen rechteckigen Abschnitt 3.1, 4.1 und einen im Wesentlichen trapezförmigen Abschnitt 3.2, 4.2.

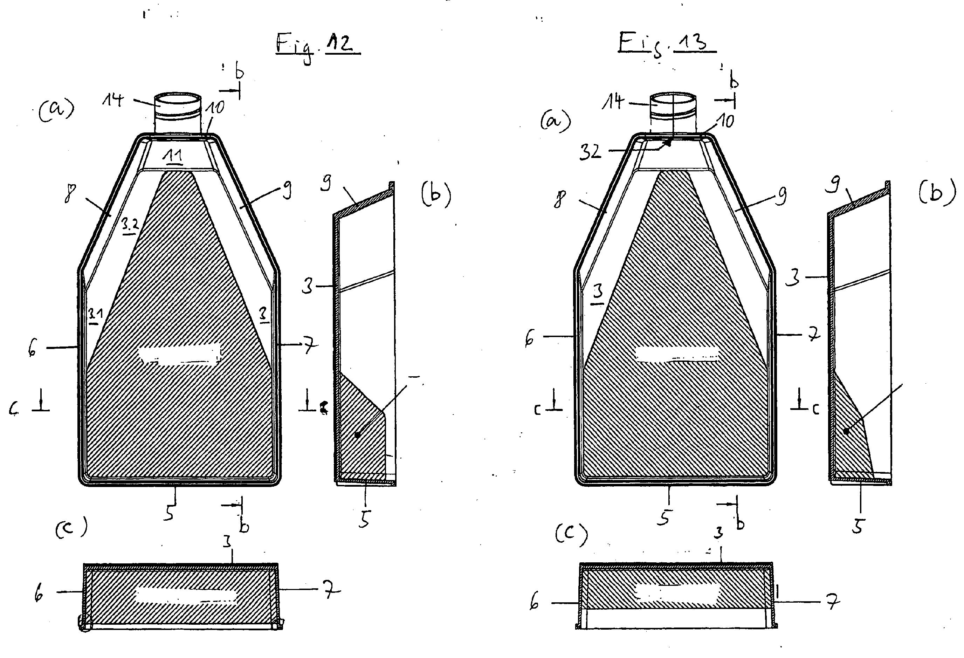

Der Abstandsbereich zwischen der Bodenwand 3 und der Deckwand 4 ist durch Seitenwände 5, 6, 7, 8, 9, 10 überbrückt, die mit den Rändern der Bodenwand 3 und der Deckwand 4 verbunden sind. Der rechteckige Abschnitt der Bodenwand 3.1 ist geringfügig kleiner als der rechteckige Abschnitt 4.1 der Deckwand 4. Infolgedessen sind die hintere Seitenwand 5 und die beiden hinteren längsseitigen Seitenwände 6, 7, welche mit den Rändern der rechteckigen Abschnitte 3.1, 4.1 verbunden sind, nur geringfügig nach außen geneigt (vgl. Fig. 12a).

Der trapezförmige Abschnitt 3.2 der Bodenwand 3 ist deutlich kleiner als der trapezförmige Abschnitt 4.2 der Deckwand 4. Infolgedessen sind die vorderen längsseitigen Seitenwände 8, 9, welche die seitlichen Ränder der trapezförmigen Abschnitte 3.2, 4.2 miteinander verbinden, stärker nach außen geneigt (vgl. Fig. 12a).

Der vordere Rand des trapezförmigen Abschnitts der Bodenwand ist mit einer trapezförmigen Schrägwand 11 verbunden. Der vordere Rand der Schrägwand 11 endet ein kleines Stück hinter dem vorderen Rand der Deckwand 4 (vgl. Fig. 12a). Zwischen diesem Rand der Schrägwand 11 und dem vorderen Rand der Deckwand 4 ist die vordere Seitenwand 10 angeordnet. Die vordere Seitenwand 10 erstreckt sich unterhalb des vorderen Randes der Schrägwand 11 bis auf das Niveau der Unterseite der Bodenwand 3 (vgl. Fig. 1, 3).

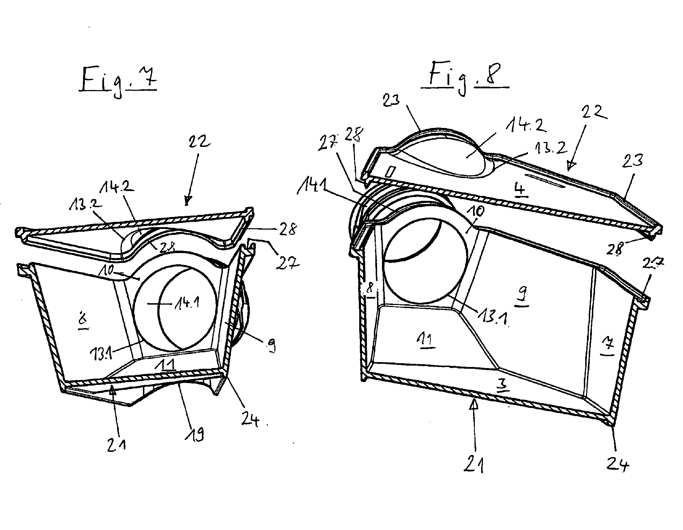

Die vorderen längsseitigen Seitenwände 8, 9 überbrücken auch den Abstand zwischen längsseitigen Rändern der Neigungsfläche 11 und den vorderen längsseitigen Rändern der Deckwand 4 (vgl. Fig. 7, 8).

Die Oberseite der Bodenwand ist eine ebene Wachstumsfläche 12.

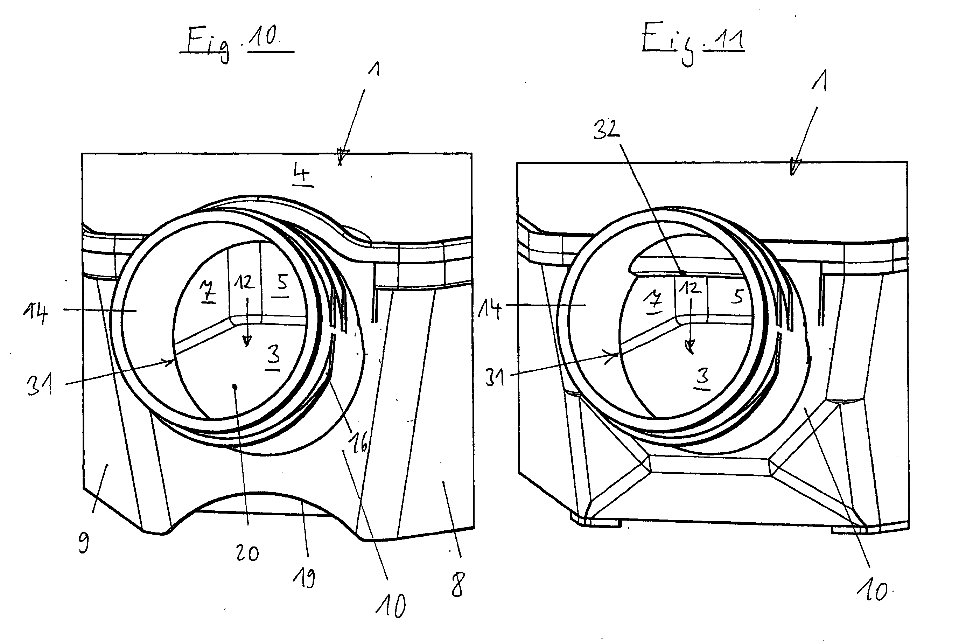

In einem an die Deckwand 4 angrenzenden Bereich der vorderen Seitenwand 10 und in dem angrenzenden Bereich der Deckwand 4 ist eine Öffnung 13 vorhanden. Ein unterer Öffnungsabschnitt 13.1 der Öffnung 13, der in der vorderen Seitenwand 10 angeordnet ist, ist oberhalb der Schrägwand 11 angeordnet. Die Öffnung 13 ist somit in vertikaler Richtung von der Bodenwand 3 beabstandet. Der untere Öffnungsabschnitt 13.1 ist in der Vorderansicht elliptisch (vgl. Fig. 10).

Ein oberer Öffnungsabschnitt 13.2, der in der Deckwand 4 angeordnet ist, erweitert sich zur vorderen Seitenwand 10 hin. Der obere Öffnungsabschnitt 13.2 hat in der Draufsicht die Form eines Abschnittes einer Ellipse (vgl. Fig. 4, 7, 8).

Ein hohlzylindrischer Flaschenhals 14 ist mit dem Rand der Öffnung 13 fest verbunden. Der Flaschenhals 14 ist spitzwinklig zur Bodenwand 3 geneigt. Am vorderen Ende steht der Flaschenhals 14 nach oben über den Abschnitt der Deckwand 4 hinaus, der parallel zur Bodenwand 3 ausgerichtet ist.

Der Flaschenhals 14 hat einen kreisförmigen Querschnitt. Er weist einen ersten Flaschenhalsteil 14.1 auf, der die Form eines Hohlzylinders hat. Der erste Flaschenhalsteil 14.1 ist an einem unteren Randabschnitt 15.1 eines stirnseitigen Randes 15 fest mit dem Rand des unteren Öffnungsabschnitts 13.1 verbunden (vgl. Fig. 3).

Ferner weist der Flaschenhals 14 einen zweiten Flaschenhalsteil 14.2 auf, der ein Hohlzylindersegment ist. Der zweite Flaschenhalsteil 14.2 ist an den seitlichen Rändern mit dem ellipsenförmigen Rand des oberen Öffnungsabschnitts 13.2 verbunden. An seinem stirnseitigen Rand ist er mit dem oberen Randabschnitt 15.2 des ersten Flaschenhalsteils 14.1 verbunden, der nach oben über die vordere Seitenwand 10 hinaussteht.

Der Flaschenhals 14 weist außen am Umfang einen Gewindegang 16 für die Verstellung eines Deckels auf.

Die vordere Seitenwand 10 hat unterhalb des Flaschenhalses eine bogenförmige Aussparung 19, die komplementär zu dem vorderen stirnseitigen Rand des zweiten Flaschenhalsteils 14.2 geformt ist.

Die Wände 3 bis 11 begrenzen eine Kulturkammer 20.

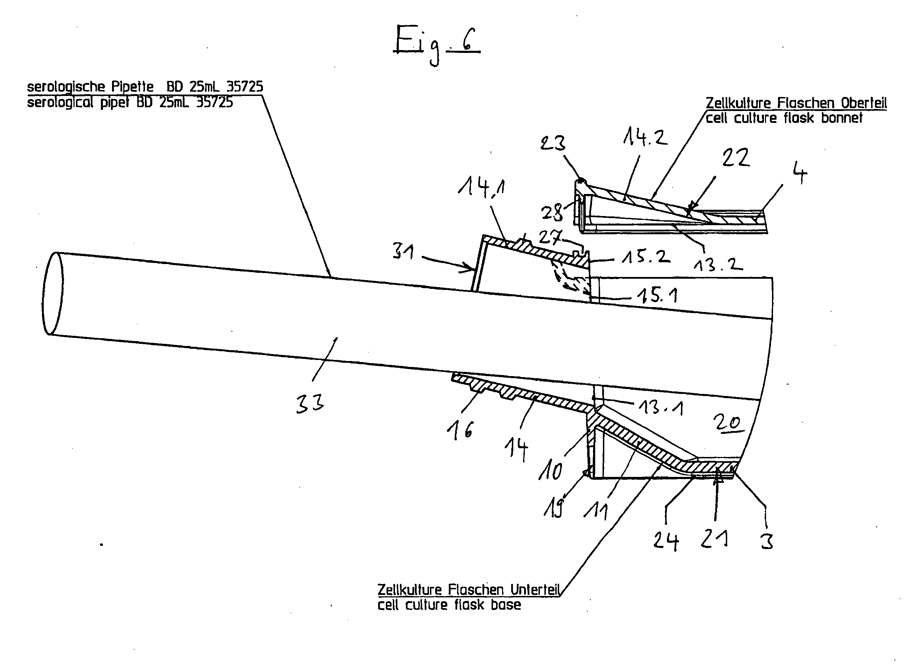

Die Bodenwand 3 sowie die Seitenwände 5 bis 10 und das erste Flaschenhalsteil 14.1 gehören zu einem Flaschenunterteil 21, das in Fig. 6 bis 8 gezeigt ist. Die Deckwand 4 und der zweite Flaschenhalsteil 14.2 gehören zu einem Flaschenoberteil 22, das in denselben Figuren gezeigt ist.

Das Flaschenoberteil 22 hat am Rand eine nach oben vorstehende, ununterbrochen umlaufende erste Rippe 23.

Vom Rand der Bodenwand 3 steht nach unten eine umlaufende zweite Rippe 24 vor.

Auf der hinteren Seitenwand 5 sind neben den seitlichen Rändern zwei nach hinten vorstehende, vertikal erstreckte hintere Rippen 25, 26 vorhanden (vgl. Fig. 5).

Das Flaschenunterteil 21 hat am oberen Rand eine nach oben geöffnete, umlaufende Nut 27 (vgl. Figuren 6 bis 7). Diese erstreckt sich entlang der oberen Ränder der hinteren sowie der längsseitigen Seitenwände 5, 6, 7, 8, 9 und neben dem ersten Flaschenhalsteil 14.1 auf dem oberen Rand der vorderen Seitenwand 10. Ferner erstreckt sich am hinteren Ende des ersten Flaschenhalsteils 14.1 über den Umfang des ersten Flaschenhalsteils 14.1 hinweg. Somit erstreckt sich die Nut 27 lediglich auf dem Umfang des ersten Flaschenhalsteils 14.1 über einen Kreisbogen und im Übrigen in einer Ebene.

Das Flaschenoberteil 22 hat umlaufend am Rand einen nach unten vorstehende Energierichtungsgeber 28. Dieser hat gemäß Fig. 6 bis 8 die Form einer Rippe mit dreieckigem Querschnitt, wobei die Spitze der Rippe nach unten weist. Der Energierichtungsgeber 28 verläuft entlang der hinteren und seitlichen Ränder der Deckwand 4 und neben dem zweiten Flaschenhalsteil 14.2 entlang des vorderen Randes der Deckwand. Ferner erstreckt er sich vorn entlang des inneren Umfangs des zweiten Flaschenhalsteils 14.2.

Der Energierichtungsgeber 28 ist senkrecht unterhalb der ersten Rippe 23 angeordnet.

Bei der Herstellung wird das Flaschenoberteil 22 auf das Flaschenunterteil 21 aufgesetzt, sodass der Energierichtungsgeber 28 in die Nut 27 eingreift. Dies ist in Fig. 2 und 3 gezeigt. Danach wird eine Sonotrode mit entsprechender Form von oben auf die erste Rippe 23 aufgesetzt. Die Sonotrode wird in Vertikalrichtung in Schwingung mit Frequenz im Ultraschallbereich versetzt. Durch die Reibung zwischen Energierichtungsgeber 28 und Grund der Nut 27 wird das Kunststoffmaterial aufgeschmolzen und miteinander verschweißt. Die Figuren 2 und 3 zeigen Flaschenoberteil 22 und Flaschenunterteil 21 in der Endlage nach dem Ultraschallschweißen, wobei die vor dem Verschweißen noch vorhandene Spitze des Energierichtungsgebers 28 in Fig. 2 und 3 eingezeichnet ist, um diesen beim Schweißen mit der Nut 27 verschmelzenden Teil des Energierichtungsgebers zu veranschaulichen.

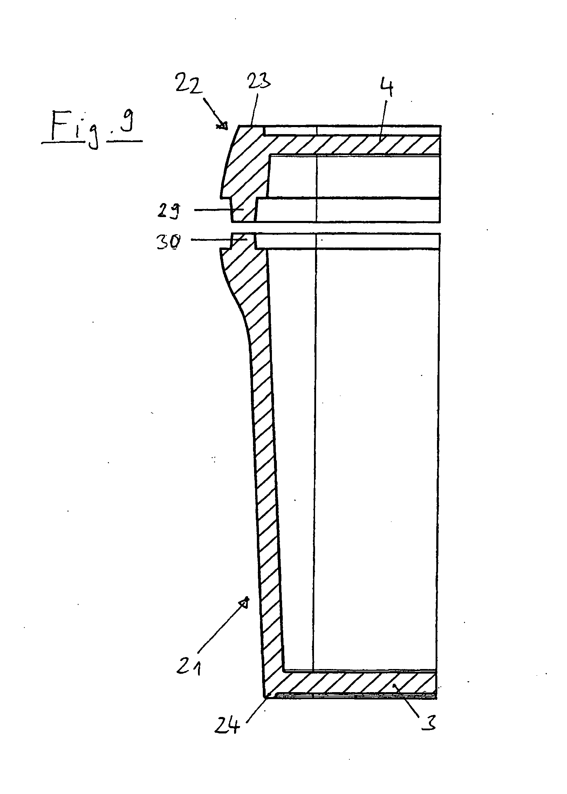

Alternativ haben das Flaschenunterteil 21 und das Flaschenoberteil 22 gemäß Fig. 9 eine Geometrie zum Herstellen einer Infrarot-Schweißverbindung. Die Geometrie umfasst eine vom Flaschenoberteil 22 umlaufend nach unten vorstehende dritte Rippe 29 und eine vom Flaschenunterteil umlaufend nach oben vorstehende vierte Rippe 30. Zum Herstellen der Verbindung wird zwischen der dritten und vierten Rippe 29, 30 ein Infrarotstrahler angeordnet und nach dem Anschmelzen der dritten und vierten Rippe 29, 30 entfernt. Danach werden die angeschmolzenen Rippen 29, 30 zusammengepresst und sind nach dem Erstarren fest miteinander verbunden.

Gemäß Fig. 10 ist bei der erfindungsgemäßen Kulturflasche 1 ein Durchgang 31 durch den Flaschenhals 14 im Bereich der Öffnung 13 nicht durch eine horizontale Kante begrenzt. Die Kulturkammer 20 ist somit gut zugänglich. Gemäß Fig. 11 behindert bei einer herkömmlichen Kulturflasche 1 eine Kante 32 den Zugang durch den Flaschenhals in die Kulturkammer 20.

Gemäß Fig. 12 ist bei der erfindungsgemäßen Kulturflasche 1 ein großer Teil der Wachstumsfläche 12 und verhältnismäßig große Teile der hinteren Sitzwand 5 und der längsseitigen Seitenwände 6, 7 mit einer serologischen Pipette 33 (vgl. Fig. 6) mit einem Dosiervolumen von 25ml erreichbar, die durch den Flaschenhals 14 von außen eingeführt ist. Gemäß Fig. 13 ist bei einer herkömmlichen Kulturflasche 1 nur ein entsprechender Teil der Wachstumsfläche 12 und ein kleinerer Teil der hinteren Seitenwand 5 und der seitlichen Seitenwände 6, 7 mittels der serologischen Pipette 33 erreichbar. Die von der Pipette 33 erreichbaren Flächen der Seitenwände 5, 6, 7 sind in Fig. 12 und 13 schraffiert. Bei der erfindungsgemäßen Kulturflasche 1 ist somit das Zuführen und Entnehmen von Zellen, Gewebe, Mikroorganismen und Nährmedium einfacher möglich als bei der herkömmlichen Kulturflasche 1.

In dem Ausführungsbeispiel von Fig. 12 und 13 sind die Kulturflaschen und die von der Pipette erreichbaren Bereiche der Wachstumsfläche maßstäblich gezeichnet. Die Kulturflasche hat im gezeichneten Beispiel die Größe 175.

### Liste der verwendeten Bezugszeichen

- 1: Kulturflasche
- 2: Flaschenkörper
- 3: Bodenwand
- 3.1: rechteckiger Abschnitt
- 3.2: trapezförmiger Abschnitt
- 4: Deckwand
- 4.1: rechteckiger Abschnitt
- 4.2: trapezförmiger Abschnitt
- 5-10: Seitenwände
- 11: Schrägwand
- 12: Wachstumsfläche
- 13: Öffnung
- 13.1: unterer Öffnungsabschnitt
- 13.2: oberer Öffnungsabschnitt
- 14: Flaschenhals
- 14.1: erster Flaschenhalsteil
- 14.2: zweiter Flaschenhalsteil
- 15: stirnseitiger Rand
- 15.1: unterer Randabschnitt
- 15.2: oberer Randabschnitt
- 16: Gewindegang
- 19: Aussparung
- 20: Kulturkammer
- 21: Flaschenunterteil
- 22: Flaschenoberteil
- 23: erste Rippe
- 24: zweite Rippe
- 25, 26: hintere Rippe
- 27: Nut
- 28: Energierichtungsgeber
- 29: dritte Rippe
- 30: vierte Rippe
- 31: Durchgang
- 32: Kante
- 33: Pipette

## Patentansprüche

1. Kulturflasche mit
• einer Kulturkammer (20) in einem Flaschenkörper (2), der eine ebene Bodenwand (3) mit einer Wachstumsfläche (12) an der Oberseite, eine ebene Deckwand (4) in einem Abstand von der Bodenwand (3) und den Abstand zwischen den Rändern der Bodenwand (3) und der Deckwand (4) überbrückende Seitenwände (5 bis 10) aufweist,
• einer Öffnung (13), die in einem Abstand von der Bodenwand (3) in aneinander angrenzenden Bereichen einer Seitenwand (10) und der ebenen Deckwand (4) vorhanden ist und einen unteren Öffnungsabschnitt (13.1) in der Seitenwand (10) und einen oberen Öffnungsabschnitt (13.2) in der Deckwand (4) hat, und
• einem hohlzylindrischen Flaschenhals (14), der mit dem Rand des unteren Öffnungsabschnittes (13.1) und dem Rand eines oberen Öffnungsabschnittes (13.2) verbunden und spitzwinklig zur Wachstumsfläche (12) ausgerichtet ist, sodass eine serologische Pipette, ein Schaber, eine Kanüle oder eine andere langgestreckte Vorrichtung zum Zugeben oder Entnehmen von Material von außen durch den Flaschenhals hindurch an die hinteren Ecken der Wachstumsfläche (12) ansetzbar ist.

2. Kulturflasche nach Anspruch 1, die ein Flaschenunterteil (21) und ein Flaschenoberteil (22) aufweist, wobei das Flaschenunterteil (21) die Bodenwand (3), die Seitenwände (5 bis 10) und ein mit einer Seitenwand (10) verbundenes erstes Flaschenhalsteil (14.1), das Flaschenoberteil (22) die Deckwand (4) und ein mit der Deckwand (4) verbundenes zweites Flaschenhalsteil (14.2) umfasst und das Flaschenunterteil (21) und das Flaschenoberteil (22) an benachbarten Rändern abdichtend miteinander verbunden sind.

3. Kulturflasche nach Anspruch 2, bei der das erste Flaschenhalsteil (14.1) ein Hohlzylinder ist, der in einem unteren Randabschnitt (15.1) eines stirnseitigen Randes (15), der in einer zur Wachstumsfläche (12) senkrechten Fläche umläuft, mit dem Rand eines unteren Öffnungsabschnitts (13.1) der Öffnung (13) in der Seitenwand (10) verbunden ist, und bei der das zweite Flaschenhalsteil (14.2) ein Hohlzylindersegment ist, welches an einem stirnseitigen Rand mit einem oberen Randabschnitt (15.2) des stirnseitigen Randes (15) des ersten Flaschenhalsteils (14.1) verbunden ist und an den beiden seitlichen Rändern mit den Rändern eines sich zum ersten Flaschenhalsteil hin erweiternden oberen Öffnungsabschnitts (13.2) der Öffnung (13) in der Deckwand (4) verbunden ist.

4. Kulturflasche nach Anspruch 2 oder 3, bei der die Verbindung zwischen Flaschenunterteil (21) und Flaschenoberteil (22) eine Ultraschall-Schweißverbindung oder eine Infrarot-Schweißverbindung oder eine Klebverbindung oder eine Rastverbindung ist.

5. Kulturflasche nach Anspruch 4, bei der die Ultraschall-Schweißverbindung zwischen Flaschenunterteil (21) und Flaschenoberteil (22) hergestellt ist durch Einschweißen eines am Rand des Flaschenoberteils (21) umlaufend nach unten vorstehenden Energierichtungsgebers (28) in eine am oberen Rand des Flaschenunterteils (21) umlaufende und nach oben offene Nut (27).

6. Kulturflasche nach einem der Ansprüche 2 bis 5, bei der das Flaschenoberteil (21) am Rand eine nach oben vorstehende, ununterbrochen oder unterbrochen umlaufende erste Rippe (23) aufweist.

7. Kulturflasche nach einem der Ansprüche 1 bis 6, bei der am Rand der Bodenwand (3) eine ununterbrochen oder unterbrochen umlaufende zweite Rippe (24) mit einer ebenen Unterseite nach unten vorsteht.

8. Kulturflasche nach einem der Ansprüche 4, 6 oder 7, bei der die Infrarot-Schweißverbindung zwischen Flaschenunterteil und Flaschenoberteil hergestellt ist durch Aufschmelzen und Zusammenpressen einer am Rand des Flaschenoberteils (22) umlaufend nach unten vorstehenden dritten Rippe (29) und einer am oberen Rand des Flaschenunterteils (21) umlaufend nach oben vorstehenden vierten Rippe (30).

9. Kulturflasche nach einem der Ansprüche 1 bis 8, bei der der Flaschenhals (14) mindestens ein Außengewinde (16) und/oder mindestens ein Schraubelement für einen Deckel aufweist.

10. Kulturflasche nach einem der Ansprüche 1 bis 9, die sich zum Flaschenhals (14) hin verjüngt.

11. Kulturflasche nach einem der Ansprüche 1 bis 10, die zwischen Bodenwand (3) und Seitenwand (10), an die der Flaschenhals angesetzt ist, eine spitzwinklig zur Bodenwand geneigte Schrägwand (11) aufweist.

12. Kulturflasche nach einem der Ansprüche 1 bis 11, bei der der Flaschenhals (14) kreiszylindrisch ist.

13. Kulturflasche nach einem der Ansprüche 1 bis 12, bei der der Übergang vom Flaschenhals (14) in die Kulturkammer (20) ganz oder teilweise glatt und/oder gerundet ist.

14. Kulturflasche nach einem der Ansprüche 1 bis 13, die deren Oberseite und Unterseite so aufeinander abgestimmt sind, dass mehrere gleiche Zellkulturflaschen (1) aufeinander stapelbar sind.

15. Kulturflasche nach einem der Ansprüche 1 bis 13, bei der die Seitenwand (10), an die der Flaschenhals (14) angesetzt ist, unterhalb des Flaschenhalses (14) eine Aussparung (19) aufweist, die geeignet ist, einen bezüglich der Deckwand (4) vorstehenden Bereich des Flaschenhalses (14) einer gleichen Kulturflasche (1) aufzunehmen, auf die die Kulturflasche (1) aufgesetzt ist.

16. Kulturflasche nach einem der Ansprüche 1 bis 15, die durch Spritzgießen hergestellt ist.

17. Kulturflasche nach einem der Ansprüche 1 bis 16, die aus Polystyrol oder einem anderen Kunststoff hergestellt ist.

## Claims

1. A culture flask comprising:
• a culture chamber (20) in a flask body (2) having an even bottom wall (3) with a growth surface (12) on the top side, an even cover wall (4) at a distance from the bottom wall (3), and side walls (5 to 10) that bridge the distance between the margins of the bottom wall (3) and the cover wall (4),
• an opening (13) that is located in adjacent regions of a side wall (10) and the even cover wall (4) at a distance from the bottom wall (3), the opening having a lower opening section (13.1) in the side wall (10) and an upper opening section (13.2) in the cover wall (4), and
• a hollow cylindrical flask neck (14) that is connected to the margin of the lower opening section (13.1) and to the margin of the upper opening section (13.2) is aligned at a sharp angle to the growth surface (12) so that a serological pipette, a scraper, a cannula or another elongated device for adding or removing material can be placed on the rear corners of the growth surface (12) from the outside through the flask neck.

2. The culture flask according to claim 1, comprising a flask base (21) and a flask bonnet (22), wherein the flask base (21) comprises the bottom wall (3), the side walls (5 to 10), and a first flask neck part (14.1) connected to a side wall (10), the flask bonnet (22) comprises the cover wall (4) and a second flask neck part (14.2) connected to the cover wall (4), and the flask base (21) and flask bonnet (22) are connected to each other in a sealing manner at adjacent margins.

3. The culture flask according to claim 2, wherein the first flask neck part (14.2) is a hollow cylinder which, in a lower margin section (15.1) of an end face margin (15) that runs in a surface perpendicular to the growth surface (12), is connected to the margin of a lower opening section (13.1) of the opening (13) in the side wall (10), and wherein the second flask neck part (14.2) is a hollow cylinder segment that is connected at an end face margin to an upper margin section (15.2) of the end face margin (15) of the first flask neck part (14.1), and is connected at the two lateral margins to the margins of an upper opening section (13.2) of the opening (13) in the cover wall (4) that expands toward the first flask neck part.

4. The culture flask according to claim 2 or 3, wherein the connection between the flask base (21) and flask bonnet (22) is an ultrasonic welded connection, or an infrared welded connection, or an adhesive connection, or a snap connection.

5. The culture flask according to claim 4, wherein the ultrasonic welded connection between the flask base (21) and flask bonnet (22) is created by welding an energy director (28) that projects downward and runs around the margin of the flask bonnet (21) into a groove (27) that is open at the top and runs around the top margin of the flask base (21).

6. The culture flask according to one of claims 2 to 5, wherein the flask bonnet (21) has an upwardly projecting, uninterrupted or interrupted peripheral first rib (23) on the margin.

7. The culture flask according to one of claims 1 to 7, wherein an uninterrupted or interrupted peripheral second rib (24) with an even bottom side projects downward from the margin of the bottom wall (3).

8. The culture flask according to one of claims 4, 6 or 7, wherein the infrared welded connection between the flask base and flask bonnet is created by melting and pressing together a downward-projecting third rib (29) running around the margin of the flask bonnet (22), and an upward-projecting fourth rib (30) running around the top margin of the flask base (21).

9. The culture flask according to one of claims 1 to 8, wherein the flask neck (14) has at least one outer thread (16) and/or at least one screw-in element for a lid.

10. The culture flask according to one of claims 1 to 9 that narrows toward the flask neck (14).

11. The culture flask according to one of claims 1 to 10 that has a sloping wall (11) angled at a sharp angle to the bottom wall between the bottom wall (3) and side wall (10) to which the flask neck is attached.

12. The culture flask according to one of claims 1 to 11, wherein the flask neck (14) is a regular cylinder.

13. The culture flask according to one of claims 1 to 12, wherein the transition from the flask neck (14) to the culture chamber (20) is completely or partially smooth and/or rounded.

14. The culture flask according to one of claims 1 to 13 with a bonnet and base that are coordinated with each other such that a plurality of equivalent cell culture flasks (1) can be stacked on each other.

15. The culture flask according to one of claims 1 to 13, wherein the side wall (10) to which the flask neck (14) is attached has, below the flask neck (14), a cut-out (19) that is suitable for accommodating a region of the flask neck (14) - projecting relative to the cover wall (4) - of an equivalent culture flask (1) on which the culture flask (1) is placed.

16. The culture flask according to one of claims 1 to 15 that is manufactured by injection molding.

17. The culture flask according to one of claims 1 to 16 that is made of polystyrene or another plastic.

## Revendications

1. Bouteille pour culture, avec
• une chambre de culture (20) dans un corps de bouteille (2), qui comporte une paroi de fond (3) plate avec une surface de croissance (12) dans la face supérieure, une paroi supérieure (4) plate en une distance de la paroi de fond (3) et des parois latérales (5 à 10) qui relient la distance entre les bords de la paroi de fond (3) et la paroi supérieure (4),
• une ouverture (13), qui existe en une distance de la paroi de fond (3) dans des zones avoisinantes d'une paroi latérale (10) et de la paroi supérieure (4) plate et qui a une partie d'ouverture inférieure (13.1) dans la paroi latérale (10) et une partie d'ouverture supérieure (13.2) dans la paroi supérieure (4), et
• un goulot (14) en forme de cylindre creux qui est relié au bord de la partie d'ouverture inférieure (13.1) et au bord d'une partie d'ouverture supérieure (13.2) et aligné en angle aigu sur la surface de croissance (12), de sorte qu'une pipette sérologique, un racleur, une canule ou un autre dispositif longitudinal pour ajouter ou prélever du matériau de l'extérieur peut être mis sur les coins arrière de la surface de croissance (12) à travers le goulot.

2. Bouteille de culture selon la revendication 1, qui comporte une partie de bouteille inférieure (21) et une partie de bouteille supérieure (22), la partie de bouteille inférieure (21) comportant la paroi de fond (3), les parois latérales (5 à 10) et une première partie de goulot (14.1) reliée à une paroi latérale (10), la partie de bouteille supérieure (22) comportant la paroi supérieure (4) et une deuxième partie de goulot (14.2) reliée à la paroi supérieure (4), la partie de bouteille inférieure (21) et là la partie de bouteille supérieure (22) étant reliées l'une à l'autre sur des bords avoisinants de manière étanche.

3. Bouteille de culture selon la revendication 2, dans laquelle la première partie de goulot (14.1) est un cylindre creux qui est relié au bord d'une partie d'ouverture inférieure (13.1) de l'ouverture (13) dans la paroi latérale (10) dans une partie de bord inférieure (15.1) d'un bord frontal (15) qui circule dans une face perpendiculaire à la surface de croissance (12), et dans laquelle la deuxième partie de goulot (14.2) est un segment d'un cylindre creux qui est relié dans un bord frontal à une partie de bord supérieure (15.2) du bord frontal (15) de la première partie de goulot (14.1), et dans les deux bords latéraux aux bords d'une partie d'ouverture supérieure (13.2) de l'ouverture (13) dans la paroi supérieure (4) qui s'élargit dans la direction vers la première partie de goulot.

4. Bouteille de culture selon les revendications 2 ou 3, dans laquelle le raccordement entre la partie de bouteille inférieure (21) et la partie de bouteille supérieure (22) est une jonction soudée par ultrason ou une jonction soudée par infrarouge ou une jonction collée ou une jonction par encliquetage.

5. Bouteille de culture selon la revendication 4, dans laquelle la jonction soudée par ultrason entre la partie de bouteille inférieure (21) et la partie de bouteille supérieure (22) est faite en soudant un capteur de direction d'énergie (28) faisant saillie vers le bas en circulant sur le bord de la partie de bouteille supérieure (22) dans une rainure (27) qui circule sur le bord supérieur de la partie de bouteille inférieure (21) et est ouverte vers le haut.

6. Bouteille de culture selon l'une quelconque des revendications 2 à 5, dans laquelle la partie de bouteille supérieure (22) comporte sur le bord une première ailette (23), faisant saillie vers le haut et circulant de manière ininterrompue ou interrompue.

7. Bouteille de culture selon l'une quelconque des revendications 1 à 6, dans laquelle une deuxième ailette (24) fait saillie vers le bas sur le bord de la paroi de fond (3) avec une face inférieure plate en circulant de manière ininterrompue ou interrompue.

8. Bouteille de culture selon l'une quelconque des revendications 4, 6 ou 7, dans laquelle la jonction soudée par infrarouge entre la partie de bouteille inférieure et la partie de bouteille supérieure est faite en fondant et pressant ensemble une troisième ailette (29) qui fait saillie vers le bas en circulant sur le bord de la partie de bouteille supérieure (22) et une quatrième ailette (30) qui fait saillie vers le haut en circulant sur le bord supérieur de la partie de bouteille inférieure (21).

9. Bouteille de culture selon l'une quelconque des revendications 1 à 8, dans laquelle le goulot (14) comporte au moins un filet extérieur (16) et/ou au moins un élément de vissage pour un couvercle.

10. Bouteille de culture selon l'une quelconque des revendications 1 à 9, qui se rajeunit en la direction du goulot (14).

11. Bouteille de culture selon l'une quelconque des revendications 1 à 10, qui comporte une palissade (11) inclinée en angle aigu sur la paroi de fond entre la paroi de fond (3) et la paroi latérale (10) sur laquelle est mis le goulot.

12. Bouteille de culture selon l'une quelconque des revendications 1 à 11, dans laquelle le goulot (14) est en forme de cylindre circulaire.

13. Bouteille de culture selon l'une quelconque des revendications 1 à 12, dans laquelle la transition du goulot (14) à la chambre de culture (20) est entièrement ou partiellement lisse et/ou arrondie.

14. Bouteille de culture selon l'une quelconque des revendications 1 à 13, dont la face supérieure et la face inférieure sont faites à correspondre de sorte que plusieurs bouteilles de culture (1) de cellules pareilles peuvent être superposées l'une au-dessus de l'autre.

15. Bouteille de culture selon l'une quelconque des revendications 1 à 13, dans laquelle la paroi latérale (10) sur laquelle le goulot (14) est mis, comporte un évidement (19) au-dessous du goulot (14) qui est apte à recevoir une zone du goulot (14) qui fait saillie par rapport à la paroi supérieure (4) d'une bouteille de culture (1) pareille au-dessus de laquelle la bouteille de culture (1) est mise.

16. Bouteille de culture selon l'une quelconque des revendications 1 à 15, qui est faite par moulage d'injection.

17. Bouteille de culture selon l'une quelconque des revendications 1 à 16, qui est faite en polystyrène ou une autre matière plastique.
